# EUROPEAN PATENT APPLICATION

(11) **EP 4 144 225 A1**
(43) Date of publication of application: **08.03.2023**
(21) Application number: 21217882.6
(22) Date of filing: 27.12.2021
(51) Int. Cl.: A23J 3/16, A23J 3/22

(54) **POROUS SCAFFOLD CONTAINING PLANT PROTEIN AND IN VITRO MEAT PREPARED USING THE SAME**

(30) Priority: 02.09.2021 KR 20210117073
(71) Applicant: Danagreen Co., Ltd., Seoul 06570 (KR)
(72) Inventor: JU, Seung Yon, 06569 Seoul (KR)
(74) Representative: Vossius & Partner Patentanwälte Rechtsanwälte mbB

(57) **Abstract**

Provided are a scaffold including a plant protein and in-vitro meat produced by using the scaffold. Considering that the scaffold consists of a plant protein, cultured muscles or adipose tissues may be ingested together with the scaffold without being separated therefrom. By adjusting a ratio of a muscle cell and an adipocyte, in-vitro meat having desirable texture may be produced, and since various types of cells may adhere, proliferate, and differentiate on the scaffold, such a scaffold may be effectively utilized for mass production of in-vitro meat.

## Description

### TECHNICAL FIELD

The present disclosure relates to a porous scaffold including a plant protein and in-vitro meat produced by using the porous scaffold.

### BACKGROUND ART

The world population is projected to reach 9 billion in 2050, and meat consumption by humans is also estimated to reach 4.65 million tons. In this regard, the Food and Agriculture Organization of the United Nations (FAO) has announced that additional production of 200 million tons of meat is necessary every year to meet the increasing demand for meat. Recently, 'in-vitro meat' is in the spotlight as a way to solve the problem of meat shortages. The term "in-vitro meat" refers to edible meat obtained by which cells are harvested from living animals and then proliferated by cell engineering technology, and is one of the cellular agriculture fields in which meat is obtained without going through a livestock rearing process.

By the development of in-vitro meat, there is a spreading awareness that some of various environmental pollution and ethical problems occurring in the process of raising and slaughtering livestock can be solved. The raising of livestock has been pointed out as a main contributor to greenhouse gas emissions. Thus, when the raising of livestock is reduced, effects such as expansion of farmland, reduction of soil erosion, and prevention of water pollution can be expected.

In addition, in-vitro meat has advantages in poor livestock environments and animal welfare related to slaughter, and can also exclude the risk of domestic animal infectious diseases, such as bovine spongiform encephalopathy and foot-and-mouth disease. Depending on a technological level, production of not only beef, but also pork, chicken, and fish is possible in vitro. Therefore, large-scale production of in-vitro meat is expected to be an effective way to save resources and reduce environmental influences occurred by the livestock industry.

Meanwhile, regarding the production cost of in-vitro meat, $ 325,000 was required to produce a single burger patty (100 g) at the beginning of the in-vitro meat development in 2013. Although the cost was reduced to $1,986/100 g as of 2017, it was still very high. In addition, due to the limitations of the production technology, the currently produced in-vitro meat has been criticized for relatively inferior taste compared to real meat. In detail, in-vitro meat is not used as an ingredient in foods of which natural taste of meat is important as in steak, but is rather used as an ingredient for a burger patty. Thus, to eliminate the difference in taste with real meat, research is actively under way to make a real meat taste by mixing in-vitro meat with muscle, oil, bones, and the like. Furthermore, considering that a cell culture medium for the production of in-vitro meat requires horse or bovine fetal serum, there is a contradiction that as the production of in-vitro meat increases, livestock slaughter also increases.

Therefore, regarding the production method for in-vitro meat, technical research to solve high cost issues, enable mass production of in-vitro meat, and give a taste similar to real meat is required.

### DESCRIPTION OF EMBODIMENTS

### TECHNICAL PROBLEM

An objective of the present disclosure is to provide a porous scaffold including a plant protein.

Another objective of the present disclosure is to provide a method of producing the porous scaffold.

Another objective of the present disclosure is to provide in-vitro meat including the porous scaffold.

Another objective of the present disclosure is to provide a method of producing in-vitro meat by using the porous scaffold.

Another objective of the present disclosure is to provide an edible product including the in-vitro meat.

### SOLUTION TO PROBLEM

An aspect of the present disclosure provides a porous scaffold including a plant-derived protein isolate, a porogen, and an emulsifier.

The porous scaffold may further include a coagulant.

The porous scaffold may be obtained by adding a plant-derived protein isolate, a porogen, and an emulsifier to a solvent to prepare a mixture, stirring the mixture, and additionally adding a coagulant to the mixture.

The term "plant-derived protein isolate" as used herein refers to a protein isolated by treating a protein present in a plant by a method commonly used in the art, and is also referred to as a plant protein.

The term "porogen" as used herein refers to any material available to produce a porous material, and is also referred to as a pore former or a pore-inducing agent. The porogen is edible, and may be one or more selected from the group consisting of agar, salt, calcium chloride, sodium carbonate, paraffin, polyethylene glycol, gelatin, and sucrose. A material that is edible and can form a pore in the plant-derived protein isolate may serve as the porogen, and may be applicable without limitation.

The term "emulsifier" as used herein refers to a material that assists mixing of two materials that are difficult to mix with each other. The emulsifier is edible, and may be one or more selected from the group consisting of glycerin, propylene glycol, monoglyceride, diglyceride, lecithin, and soybean phospholipid. A material that is edible and can assist mixing of the plant-derived protein isolate with the porogen may serve as the emulsifier, and may be applicable without limitation.

The term "solvent" as used herein refers to a material that dissolves a solute. The solvent is edible, and may be one or more selected from the group consisting of glycerin, propylene glycol, monoglyceride, diglyceride, lecithin, and soybean phospholipid.

The emulsifier and the solvent may be the same materials. Any material may serve as the emulsifier and the solvent at the same time.

The coagulant is edible, and may be one or more selected from the group consisting of glucono-delta-lactone, calcium chloride, calcium sulfate, and magnesium chloride.

The porous scaffold may have any one or more properties selected from (a) to (c) below:
(a) compressive strength in a range of 80 kPa to 150 kPa;
(b) tensile strength in a range of 40 gf to 70 gf, yield strength in a range of 18 kPA to 32 kPA, and yield elongation in a range of 10 % to 20 %; and
(c) hardness in a range of 0.4 kgf to 1.0 kgf, cohesiveness in a range of 0.7 to 0.9, springiness in a range of 0.8 to 1.2, gumminess in a range of 0.4 to 0.6, and chewiness in a range of 0.3 to 0.7.

In detail, the porous scaffold may have compressive strength in a range of about 80 kPa to about 150 kPa, about 90 kPa to about 140 kPa, about 95 kPa to about 135 kPa, or about 100 kPa to about 130 kPa. The porous scaffold may have: tensile strength in a range of about 40 gf to about 70 gf, about 45 gf to about 65 gf, or about 50 gf to about 65 gf; yield strength in a range of about 18 kPA to about 32 kPA, about 20 kPA to about 31 kPA, or about 22 kPA to about 30 kPA; and yield elongation in a range of about 10 % to about 20 %, about 12 % to about 18 %, or about 13 % to about 17 %. The porous scaffold may have: hardness in a range of about 0.4 kgf to about 1.0 kgf, about 0.5 kgf to about 0.9 kgf, or about 0.6 kgf to about 0.7 kgf; cohesiveness in a range of about 0.6 to about 1.0 or about 0.7 to about 0.9; springiness in a range of about 0.8 to about 1.2 or about 0.9 to about 1.1; gumminess in a range of about 0.3 to about 0.7, about 0.4 to about 0.6, or about 0.5 to about 0.6; and chewiness in a range of about 0.3 to about 0.7, about 0.4 to about 0.6, or about 0.5 to about 0.6.

In an embodiment, as a result of performing a compression test on the porous scaffold, the compressive strength may be measured to be about 116 kPa in average. In addition, as a result of performing a tensile test on the porous scaffold, the tensile strength may be measured to be about 56 gf in average, the yield strength may be measured to be about 26 kPA in average, and the yield elongation may be measured to be about 15 % in average. In addition, as a result of performing texture profile analysis (TPA) on the porous scaffold, the hardness may be measured to be about 0.67 kgf in average, the adhesiveness may be measured to be about 0.002 kgf*sec in average, the cohesiveness may be measured to be about 0.8 in average, the springiness may be measured to be about 1.0 in average, the gumminess may be measured to be about 0.6 in average, the chewiness may be measured to be about 0.5 in average, the resilience may be measured to be about 0.6 in average, and the brittleness may be measured to be about 0.67 kgf in average.

The porous scaffold may have any one or more properties selected from (d), (e), and (o) below:
(d) open porosity in a range of 40 % to 80 %;
(e) total porosity in a range of 40 % to 80 %; and
(o) pore interconnectivity in a range of 80 % to 99.9 %.

In detail, the porous scaffold may have open porosity in a range of about 40 % to about 80 %, about 50 % to about 70 %, about 55 % to about 65 %, or about 55 % to about 60 %. The porous scaffold may have total porosity in a range of about 40 % to about 80 %, about 50 % to about 70 %, about 55 % to about 65 %, or about 55 % to about 60 %. The porous scaffold may have pore interconnectivity in a range of about 80 % to about 99.9 %, about 85 % to about 99.9 %, about 90 % to about 99.9 %, or about 95 % to about 99.9 %.

In an embodiment, as a result of analyzing morphological properties of the porous scaffold, the open porosity may be measured to be about 59 %, the total porosity may be measured to be about 59 %, and the pore interconnectivity may be measured to be about 99 %.

The porous scaffold may have a pore size in a range of about 10 µm to about 600 µm, 20 µm to about 550 µm, about 30 µm to about 500 µm, about 40 µm to about 450 µm, about 50 µm to about 400 µm, about 60 µm to about 350 µm, about 70 µm to about 300 µm, about 75 µm to about 250 µm, about 80 µm to about 200 µm, about 85 µm to about 150 µm, about 70 µm to about 130 µm, about 80 µm to about 120 µm, about 90 µm to about 110 µm, about 50 µm to about 600 µm, about 50 µm to about 400 µm, about 50 µm to about 300 µm, about 50 µm to about 250 µm, about 50 µm to about 200 µm, or about 50 µm to about 150 µm.

The porous scaffold may have elasticity in a range of about 1 kPa to about 10 kPa, about 2 kPa to about 8 kPa, about 3 kPa to about 8 kPa, about 1 kPa to about 5 kPa, about 4 kPa to about 10 kPa, about 6 kPa to about 10 kPa, about 1 kPa to about 3 kPa, or about 4 kPa to about 6 kPa.

The porous scaffold may undergo a reversible phase transition to a solid state or a semi-solid state. In addition, the porous scaffold may be insoluble.

The plant protein may be isolated from at least one selected from the group consisting of nuts, beans, soybeans, mung beans, kidney beans, peas, mushrooms, vegetables, grains, and a combination thereof.

In an embodiment, the porous scaffold may be produced by using an isolated soy protein (ISP).

Cells may be dispensed in the porous scaffold.

Use of the porous scaffold may include three-dimensional cell culture.

In an embodiment, various types of cells may adhere, proliferate, or differentiate on the porous scaffold.

In an embodiment, adipose-derived stem cells, muscle cells, or adipocytes may be dispensed in the porous scaffold and cultured three-dimensionally.

The porous scaffold may have effects of not only stably and continuously culturing cells, but also exhibiting a culture pattern suitable for the cell properties. In addition, the porous scaffold according to an embodiment may have effects of enabling long-term culture even by the existing 2D culture methods of culturing cells in a cell culture dish or plate.

The cells dispersed in the porous scaffold may include cells selected from the group consisting of bacterial cells, yeast cells, mammalian cells, insect cells, and plant cells.

The cells may include, for example: eukaryotic cells from yeast, fungi, protozoa, plants, higher plants, and insects; cells from amphibians; or mammalian cells such as CHO, HeLa, HEK293, and COS-1. For example, the cells may include, as commonly used in the art, in vitro cells, graft cells, primary cell cultures (e.g., in vitro and ex vivo cell cultures), in vivo cells, and mammalian cells including human cells. In addition, an organism may be a yeast, a fungus, a protozoan, a plant, a higher plant, an insect, an amphibian, or a mammal.

The cells dispersed in the porous scaffold may include one selected from: cartilage cells; fibrocartilage cells; osteocytes; osteoblasts; osteoclasts; synovial cells; marrow cells; nerve cells; mesenchymal cells; epithelial cells; hepatocytes; muscle cells; stromal cells; vascular cells; stem cells; embryonic stem cells; mesenchymal stem cells; adipocytes; precursor cells derived from adipose tissues; peripheral blood precursor cells; stem cells isolated from adult tissues; induced pluripotent stem cells (iPS cells), tumor cells derived from the cells above, and a combination thereof. In detail, the cells dispersed in the porous scaffold may be stem cells, adipocytes, precursor cells derived from adipose tissues, or muscle cells.

To induce differentiation or proliferation of the dispersed cells, the porous scaffold may further include a differentiation-inducing factor, a growth factor, a growth stimulator, a physiologically active material, or a cell-binding mediator such as a peptide or a polysaccharide.

In an embodiment, the growth factor or the growth stimulator may include: a cell adhesion mediator; a biologically active ligand; an integrin-binding sequence; a ligand; various growth and/or differentiation agents (e.g., a epithelial cell growth factor, IGF-I, IGF-II, TGF-[beta]), a growth and differentiation factor; a stromal-derived factor (SDF-1); a vascular endothelial growth factor; a fibroblast growth factor; a platelet-derived growth factor; an insulin-derived growth factor; a transforming growth factor; a parathyroid hormone; a parathyroid hormone-related peptide, bFGF; a nerve growth factor (NGF) or a muscle morphogenic factor (MMF); a heparin-binding growth factor (HBGF), a transforming growth factor alpha or beta, alpha fibroblast growth factor (FGF), epidermal growth factor (TGF->EGF?), vascular endothelial growth factor (VEGF), SDF-1; TGF[beta] superfamily factor; BMP-2; BMP-4; BMP-6; BMP-12; sonic hedgehog; GDF5; GDF6; GDF8; PDGF); small molecules affecting up-regulation of specific growth factors; tenascin-C; hyaluronic acid; chondroitin sulfate; fibronectin; decorin; thromboplastin; thrombin-derived peptides; TNF alpha/beta; matrix metalloproteinase (MMP); a heparin-binding domain; heparin; heparan sulfate; or DNA fragment or DNA plasmid thereof, short interfering RNA (siRNA), a trait infectious agent, or a mixture thereof.

Another aspect of the present disclosure provides a method of producing the porous scaffold, the method including: adding a plant-derived protein isolate, a porogen, and an emulsifier to a solvent to prepare a mixture; stirring the mixture to produce a stirred mixture; and adding a coagulant to the stirred mixture to form a scaffold.

The plant-derived protein isolate, the porogen, the emulsifier, the solvent, and the coagulant may respectively be the same as described above.

The stirring of the mixture may be performed by stirring at a speed in a range of 30 rpm to 150 rpm, 40 rpm to 120 rpm, 50 rpm to 100 rpm, or 50 rpm to 70 rpm, or for example, at a speed of 60 rpm, for 3 minutes to 60 minutes, 5 minutes to 50 minutes, 5 minutes to 40 minutes, 5 minutes to 30 minutes, or 5 minutes to 20 minutes, or for example, for 10 minutes.

The method may further include, before the adding of the coagulant, steps of molding and freeze-drying the mixture. The molding may be performed in a casting mold having a desired shape and size. The freeze-drying may include: freezing at a temperature in a range of -40 °C to -240 °C, -50 °C to -200 °C, -60 °C to -160 °C, -70 °C to -120 °C, -60 °C to -100 °C, or -70 °C to -90 °C, or for example, -80 °C, for 10 minutes to 3 hours, 30 minutes to 2 hours, or 30 minutes to 1 and half hour, or for example, for 1 hour; and a drying step.

In detail, the freeze-drying may include: after coating the entire surface of a frozen plant protein composition with sodium bicarbonate, an adsorption step at room temperature for 1 hour to 10 hours, 2 hours to 8 hours, 3 hours to 6 hours, or 4 hours to 5 hours, or for example, for 4 hours; and then a drying step at room temperature for 1 hour to 10 hours, 2 hours to 8 hours, 3 hours to 6 hours, or 4 hours to 5 hours, or for example, for 4 hours. The sodium bicarbonate may serve to facilitate the flow of the coagulant to the inside of the plant protein composition by promoting the formation of pores on the surface of the composition. When the coagulant is added to the frozen plant protein composition without coating the sodium bicarbonate therewith, the coagulant may first act on the surface of the composition so that pores may be irregularly formed therein, thereby producing a structure unsuitable for culturing the cells.

In addition, the method of the present disclosure may further include washing the coagulated plant protein composition with distilled water or an organic solvent (for example, ethanol) and performing autoclave thereon, to remove impurities other than proteins.

Another aspect of the present disclosure provides in-vitro meat including: the porous scaffold; and the cells dispersed in the porous scaffold.

The porous scaffold may be the same as described above.

The term "in-vitro meat (also referred to as cultured meat, synthetic meat, cell-cultured meat, clean meat, or vat meat) refers to lean meat produced by cell-engineering techniques in which live animal cells are cultured to culture meat without livestock farming. In-vitro meat is also referred to as artificial meat. Such in-vitro meat is not yet commercially produced, and several studies and projects are currently developing in-vitro meat techniques experimentally.

Cytodex 1^{®} microcarrier previously known in the art to be used for the production of in-vitro meat is compared with the porous scaffold including the plant protein according to an embodiment, and the results of property comparison are shown in Table 1.

**[Table 1]**

| | Cytodex 1^{®} | Embodiment of the present disclosure |
|---|---|---|
| Manufacturer | Cytiva (GE Healthcare) | DaNAgreen |
| Main ingredient | dextran | isolated soy protein |
| Main characteristics | (+)-surface charqe | multi porous |
| Shape | spherical microcarrier | sponge-like sheet |
| Size | ø190 µm | 25 × 25 × 2 mm |
| Max cell no. per qram | 24 M | 100 M |
| Scaffold no. per qram | 172000 | 5 |
| Max cell no. per scaffold | 140 | 20 M |
| Cells attachment rate | -60% | 100% |
| Price per 1kg meat | $16,340 | $6.52 |
| Price ratio | 2506.1 | 1.0 |

The information on Cytodex 1^{®} is obtained from the prior art document [Cytotechnology 2018 Apr; 70(2):503-512] and the website of the manufacturer (www.cytivalifesciences.com). The properties obtained in an embodiment are not limited to the entire disclosure, but are limited to the porous scaffold produced according to the embodiment. Another aspect of the present disclosure provides a method of producing in-vitro meat, the method including dispensing cells on the porous scaffold; culturing the cell three-dimensionally; and forming a three-dimensional cell aggregate.

The cells may include one or more selected from the group consisting of stem cells, muscle cells, and adipocytes, but are not limited thereto.

In an embodiment, in-vitro meat may be produced by dispensing muscle cells or adipocytes on the porous scaffold, and culturing the cells three-dimensionally. In addition, since the porous scaffold consists of edible materials, in-vitro meat may be produced without any step of isolating the cultured cells from the porous scaffold.

In an embodiment, in-vitro meat may be produced by dispensing stem cells in the porous scaffold and culturing the cells to be differentiated into muscles or adipose tissues. In addition, since the porous scaffold consists of edible materials, in-vitro meat may be directly ingested without the need to separate the differentiated muscles or adipose tissues from the scaffold.

Another aspect of the present disclosure provides a method of producing in-vitro meat, the method including: dispensing a cells on the porous scaffold; culturing the cells three-dimensionally; and forming a three-dimensional cell aggregate.

In an embodiment, in-vitro meat may be produced by the one-step process in which the cells and the porous scaffold are put into a bioreactor. When the cells and the porous scaffold are simultaneously put into a single incubator, the adhesion, proliferation, and differentiation of the cells occur at once, thereby constructing a mass production system.

Another aspect of the present disclosure provides in-vitro meat produced by the method disclosed herein, the in-vitro meat including the porous scaffold.

Another aspect of the present disclosure provides an edible product including the in-vitro meat.

The porous scaffold and the in-vitro meat may respectively be the same as described above.

Overlapping contents may be omitted in consideration of the complexity of the present disclosure. Unless specifically defined otherwise in the present specification, terms may have meanings commonly used in the art to which the present disclosure pertains.

### ADVANTAGEOUS EFFECTS OF DISCLOSURE

A scaffold including a plant protein according to an embodiment and in-vitro meat produced by using the scaffold consist of a plant protein, and thus cultured muscles or adipose tissues may be ingested together with the scaffold without being separated therefrom. By adjusting a ratio of muscle cells and adipocytes, in-vitro meat having desirable texture may be produced, and since various types of cells may adhere, proliferate, and differentiate on the scaffold, such a scaffold may be effectively utilized for mass production of in-vitro meat.

### BRIEF DESCRIPTION OF DRAWINGS

FIG. 1 is a photograph of a porous scaffold produced according to an embodiment, the porous scaffold including a plant-derived protein isolate.
FIG. 2 is a stress-strain curve shown in the process of measuring compressive strength of the porous scaffold produced according to an embodiment.
FIG. 3 is a stress-strain curve shown in the process of measuring tensile strength of the porous scaffold produced according to an embodiment.
FIG. 4 is a curve showing the results of texture profile analysis (TPA) performed on the porous scaffold produced according to an embodiment.
FIGS. 5 to 8 show the shapes of scaffolds that are previously known for cell culture, wherein FIG. 5 shows the shape of a concave microwell; FIG. 6 shows the shape of a porous scaffold; FIG. 7 shows the shape of a non-porous microbead; and FIG. 8 shows the shape of a porous microbead.
FIG. 9 is a photograph obtained by staining adipose-derived stem cells on the 9th day and the 15th day of cell culture after the cells are dispensed in the porous scaffold produced according to an embodiment.
FIG. 10 is a schematic diagram illustrating a process of producing in-vitro meat in a bioreactor by using the porous scaffold produced according to an embodiment.
FIG. 11 shows the state of culturing in-vitro meat, in-vitro chicken, and in-vitro pork by using the porous scaffold produced according to an embodiment.
FIG. 12 is a photograph showing the results of live/dead assay performed on the 13th day and the 20th day in the process of culturing bovine muscle cells by using the porous scaffold produced according to an embodiment.
FIG. 13 is a photograph of the scaffold before the cell culture (left) and the scaffold at week 4 of the cell culture (right) in the process of culturing bovine muscle cells by using the porous scaffold produced according to an embodiment.
FIG. 14 is a photograph obtained by staining bovine muscle with Calcein-AM/Desmin after the cells were cultured for 7 days by using the porous scaffold produced according to an embodiment.
FIG. 15 is a photograph obtained by staining bovine muscle cells with Phalloidin/Desmin after the cells were cultured for 7 days by using the porous scaffold produced according to an embodiment.
FIG. 16 is a photograph showing the results of live/dead assay performed on chicken cardiomyocytes, wherein the cells were first cultured by using the porous scaffold produced according to an embodiment in a 10 % DMEM culture medium for 7 days, and then, cultured for another 7 days after the culture medium was replaced with a 2 % DMEM culture medium.
FIG. 17 is a photograph showing the results of live/dead assay performed on chicken cardiomyocytes after the cells were cultured by using the porous scaffold produced according to an embodiment in a 10 % DMEM culture medium for 14 days.
FIG. 18 is a photograph showing the results of live/dead assay performed on chicken cardiomyocytes after the cells were cultured by using the porous scaffold produced according to an embodiment in a 2 % DMEM culture medium for 14 days.
FIG. 19 is a photograph showing the results of live/dead assay performed on the 13th day, the 17th day, and the 38th day in the process of culturing silki muscle cells by using the porous scaffold produced according to an embodiment.
FIG. 20 is a photograph showing the results of live/dead assay performed on the 14th day in the process of culturing bovine muscles cells and on the 8th day in the process of culturing chicken muscle cells, wherein the culturing was performed by using the porous scaffold produced according to an embodiment.

### MODE OF DISCLOSURE

Hereinafter, preferable examples are provided to help understanding of the present disclosure. However, the following examples are only provided for easy understanding of the present disclosure, and the contents of the present disclosure are not limited by the following examples.

### Example 1. Production of porous scaffold including plant-derived protein isolate

A porous scaffold including a plant-derived protein isolate was produced as follows.

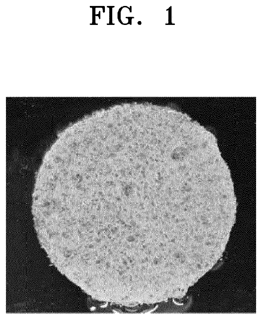

10 g of powdered isolated soy protein (ISP) and 2 g of agar were added to 10 ml of edible glycerin (0409, ES food ingredient) and mixed in a steam-stirring machine at a speed of 60 rpm for 10 minutes (D-201, Daeduck Machinery) to produce a mixture. Then, 30 ml of sterile deionized water was added to the mixture, and stirred at a speed of 5,000 rpm for 10 minutes by using a high-speed stirrer (MaXtir^{™} 500S, DAIHAN-brand^{®}). Afterwards, the resultant mixture was cast to a mold (100 mm × 100 mm × 2 mm) to form a desired shape, and then frozen at -80 °C for 1 hour to prepare a frozen sample. Baking soda was sufficiently applied to all surfaces of the frozen sample, allowed for adsorption at room temperature for 4 hours, and dried at 60 °C for 4 hours. After the dried sample was added to 1 L of sterile deionized water, 2 g of glucono-delta-lactone (E575, JUNGBUNZLAUER S.A) was added thereto for coagulation at 80 °C for 2 hours. Afterwards, the coagulated sample was washed 5 times, each with 100 % ethanol and sterile deionized water at room temperature, placed in 1 L of sterile deionized water, and
subjected to high-pressure sterilization twice to remove impurities other than proteins, thereby producing a porous scaffold including plant protein components. FIG. 1 is a photograph of the porous scaffold produced in this example.

All materials for producing the porous scaffold are resources approved as food, and thus the porous scaffold can be ingested.

### Example 2. Characterization of porous scaffold

### Example 2.1 Morphological characterization of porous scaffold

The morphological properties of the porous scaffold of Example 1 were evaluated by using SKYSCAN1272 ex-vivo micro-CT (Bruker microCT, Belgium) under the following conditions:
- X-ray source : 40 kV, 200 uA, No-filter, rotation step 0.15°;
- Resolution: 1 um pixel resolution; and
- Staining with 0.1 % iodine-potassium iodide (IKI) overnight.

Section creation was analyzed by using a NRecon software, section rotation was analyzed by using a DataViewer software, analysis was performed by using a CTAn software, volume rendering creation was analyzed by using a CTVox software, and surface rendering was analyzed by using a CTAn+CTVol software, and the results are shown in Table 2.

**[Table 2]**

| | Abbreviation | Measured value | Unit |
|---|---|---|---|
| Percent objective volume | Obj.V/TV | 40.91257477 | % |
| Structure thickness | St.Th | 0.02461021 | mm |
| Structure separation | St.Sp | 0.09660629 | mm |
| Open porosity | Po(op) | 58.55148608 | % |
| Total porosity | Po(tot) | 59.08742523 | % |
| Pore interconnectivity | | 58.55/59.09 = 99.1 | % |

| | | | |
|---|---|---|---|
| - Percent objective volume: volume ratio occupied by the scaffold. - Structure thickness: average spacing between pores. - Structure separation: average size of pores. - Open porosity: ratio of pores connected to the outside to total volume of the scaffold. - Total porosity: ratio of pores to total volume of the scaffold. - Pore interconnectivity: ratio of pores connected to the outside to the total pores. | | | |

Since the scaffold of Example 1 had the pore interconnectivity of about 99 %, the movement of the cells and the flow of the culture medium were smooth, and thus it was confirmed that the scaffold of Example 1 was suitable for the growth and differentiation of various cells.

### Example 2.2 Physical characterization of porous scaffold

To analyze physical properties of the scaffold of Example 1, the compressive strength and the tensile strength were measured by using a TXA^{™} texture analyzer (manufactured by Yeonjin S-Tech Corp.) as follows, and Texture profile analysis (TPA) was also performed.

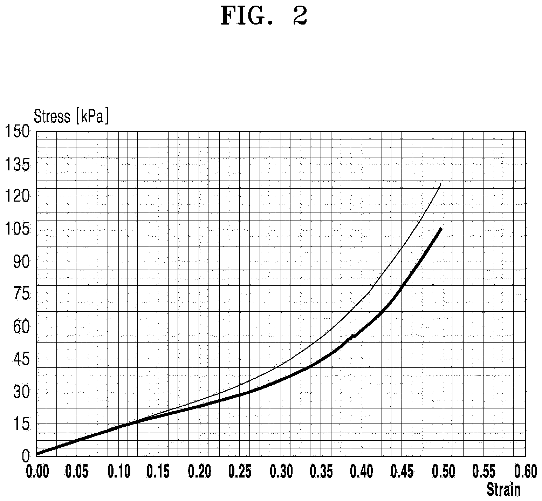

The compressive strength was measured according to the Compression Test protocol. In detail, a sample was placed on a plate of a texture analyzer equipped with a compression fixation device, and compressed with a square probe (10 mm × 10 mm). Then, the initial strain rate was set to be 0.1[1/s] and 0.033[1/s] for measurement. The measurement results are shown in Table 3 and FIG. 2.

**[Table 3]**

| | | | | | | |
|---|---|---|---|---|---|---|
| Testing Method | | Compression Test | | | | |
| Temperature | | 21.5 degree Celsius | | | | |
| Humidity | | Ambient | | | | |
| Load Cell | | 30 kgf | | | | |

| | Width mm | Length mm | Thickness mm | speed mm/s | Max Load gf | Max Stress kPa |
|---|---|---|---|---|---|---|
| 0.1 strain rate | 7.765 | 7.260 | 1.778 | 0.178 | 724.368 | 126.009 |
| 0.033 strain rate | 7.726 | 7.650 | 1.689 | 0.051 | 637.388 | 105.756 |
| A veraqe | 7.746 | 7.455 | 1.734 | 0.114 | 680.878 | 115.882 |
| Standard deviation | 0.028 | 0.276 | 0.063 | 0.090 | 61.504 | 14.320 |
| Standard deviation(%) | 0.4 | 3.7 | 3.6 | 78.7 | 9.0 | 12.4 |

Referring to Table 3 and FIG. 2 and as being measured in a way that the sample was placed on the plate of the texture analyzer and compressed with the square probe (10 mm × 10 mm) with the initial strain rate set to be 0.1[1/s] and 0.033[1/s] for the measurement, the porous scaffold according to an embodiment had the compression strength with the average width of 7.746 mm, the average length of 7.455 mm, the average thickness of 1.734 mm, the seismic wave speed of 0.114 mm/s , and the maximum stress of 115.882 kPa, in the case of the maximum load of 680.878 gf.
- Seismic wave speed: speed at which an objective deformed by an external force attempts to return to the original shape when the force is removed.
- Maximum stress: (also referred to as compression strength) maximum compressive stress that an objective can withstand without breaking.

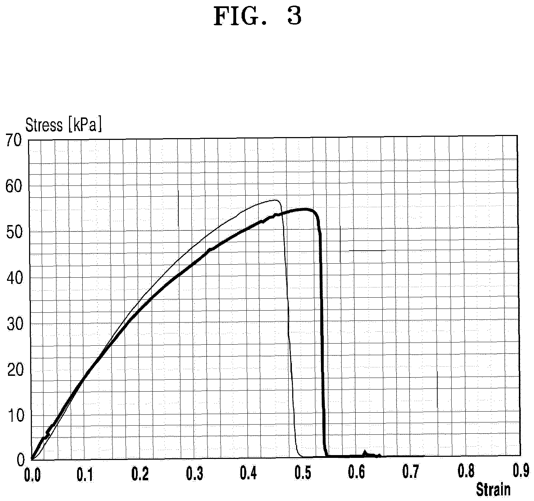

Here, the tensile strength was measured by gripping the sample to tensile grips of the texture analyzer and setting the initial strain rates to be 0.1[1/s] and 0.033[1/s] for measurement. The measurement results are shown in Table 4 and FIG. 3.

**[Table 4]**

| | | | | | | | | |
|---|---|---|---|---|---|---|---|---|
| Testing Method | | Tensile Test | | | | | | |
| Temperature | | 21.5 degree Celsius | | | | | | |
| Humidity | | Ambient | | | | | | |
| Load Cell | | 30 kgf | | | | | | |

| | Width mm | Thickness mm | Length mm | speed mm/s | Yield Load gf | Yield Stress kPa | Yield Elongation % | Max Load gf |
|---|---|---|---|---|---|---|---|---|
| 0.1 strain rate | 14.000 | 1.500 | 20.000 | 2.033 | 60.209 | 28.117 | 16.000 | 120.820 |
| 0.033 strain rate | 14.000 | 1.500 | 20.000 | 0.617 | 52.489 | 24.511 | 14.297 | 116.659 |
| A veraqe | 14.000 | 1.500 | 20.000 | 1.325 | 56.349 | 26.314 | 15.148 | 118.739 |
| Standard deviation | 0.000 | 0.000 | 0.000 | 1.001 | 5.459 | 2.549 | 1.204 | 2.942 |
| Standard deviation(%) | 0 | 0.0 | 0.0 | 75.5 | 9.7 | 9.7 | 7.9 | 2.5 |

Referring to Table 4 and FIG. 3 and as being measured in a way that the sample was gripped to the tensile grips of the texture analyzer and the initial strain rate was set to be 0.1[1/s] and 0.033[1/s] for the measurement, the porous scaffold according to an embodiment had the tensile strength with the average width of 14.000 mm, the average length of 20.000 mm, the average thickness of 1.500 mm, the seismic speed of 1.325 mm/s, the yield load of 56.348 gf, the yield stress of 26.314 kPA, and the yield elongation of 15.148 %, in the case of the maximum load of 118.739 gf.
- Yield load: (also referred to as tensile strength) force immediately before breaking an objective .
- Yield strength (or yield strength): when an external force is applied to an objective to pull it in opposite directions, the length of the objective increases. To some extent of the force, the lengthened objective returns to the original size when the external force is removed. When force greater than some extent of the force is applied, the objective cannot return to the original state and the length of the objective further increases. Here, the maximum force at which the objective can return to the original state is called yield stress or yield strength.
- Yield elongation: Change in the length of an objective until deformation increases under almost constant stress state and the stress subsequently begins to increase smoothly, as expressed in a percentage with respect to the original length of objective .

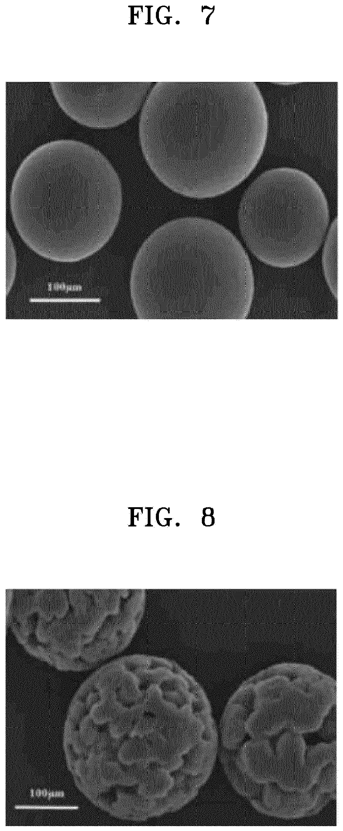

Here, the TPA was measured in a way that a sample was placed on a plate of a texture analyzer and compressed by 50 % twice with a cylinder probe having a diameter of 25.4 mm at a speed of 60 mm/min. The measurement was repeated three times, and the results are shown in Table 5 and FIG. 7.

**[Table 5]**

| | W idt h | T hi ck n es s | Cr oss - sec tio nal are a S. A | Po int dis tan ce G. L | Te st sp ee d | Har dne ss | Adhe siven ess | Cohe siven ess | Sprin gine ss | Gu mmi nes s | Che win ess | Resi lienc e | Brittl enes s |
|---|---|---|---|---|---|---|---|---|---|---|---|---|---|
| | m m | m m | m m² | m m | m m/ mi n | kgf | kgf*s ec | - | - | - | - | - | kgf |
| TPA _1 | 1 4. 7 0 | 1 2. 7 0 | 18 6.6 9 | 7.6 0 | 30 0.0 0 | 0.6 729 | 0.002 7 | 0.81 | 0.99 | 0.5 5 | 0.5 4 | 0.55 | 0.67 29 |
| TPA _2 | 1 4. 2 0 | 1 3. 7 0 | 19 4.5 4 | 7.6 4 | 30 0.0 0 | 0.6 418 | 0.001 3 | 0.82 | 1.02 | 0.5 3 | 0.5 4 | 0.55 | 0.64 17 |
| TPA _3 | 1 4. 0 0 | 1 4. 5 0 | 20 3.0 0 | 7.5 0 | 30 0.0 0 | 0.6 922 | 0.002 9 | 0.81 | 0.98 | 0.5 6 | 0.5 5 | 0.54 | 0.69 21 |
| Aver age | 1 4. 3 0 | 1 3. 6 3 | 19 4.7 4 | 7.5 8 | 0.0 0 | 0.6 689 | 0.002 3 | 0.82 | 1.00 | 0.5 5 | 0.5 4 | 0.55 | 0.66 89 |
| Stan d.De vi | 0. 3 6 | 0. 9 0 | 8.1 6 | 0.0 7 | 0.0 0 | 1.6 388 | 0.005 7 | 2.00 | 2.44 | 1.3 4 | 1.3 3 | 1.34 | 1.63 87 |

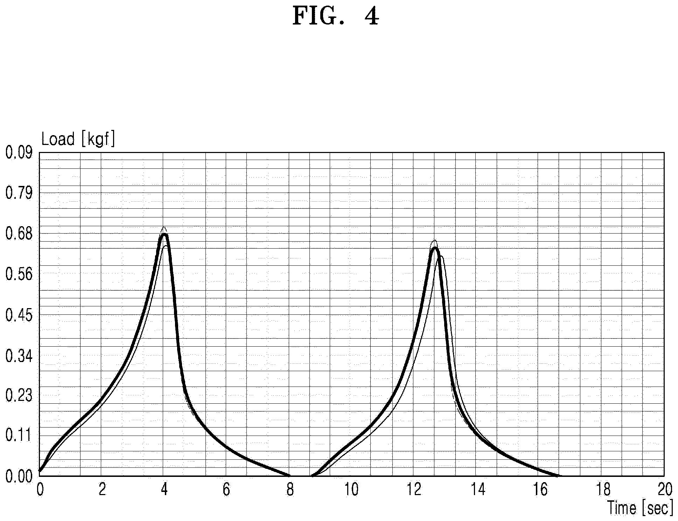

Referring to Table 5 and FIG. 4 and as being measured in a way that a sample was placed on a plate of the texture analyzer, compressed by 50 % with a cylinder probe having a diameter of 25.4 mm at a speed of 60 mm/min, the physical properties of the porous scaffold according to an embodiment include, in average, the hardness of 0.6689 kgf, the adhesiveness of 0.0023 kgf*sec, the cohesiveness of 0.82, the springiness of 1.00, the gumminess of 0.55, the chewiness of 0.54, the resilience of 0.55, and the brittleness of 0.6689 kgf.
- Hardness: Force required to reach desired deformation.
- Adhesiveness: Force required to separate an objective from a probe.
- Cohesiveness: Force to sustain the shape of an objective as it is. In case of cohesiveness greater than adhesiveness, a sample does not stick to a probe.
- Springiness (also referred to as elasticity): Property in which a sample deformed by compression attempts to return to the original state after the force is removed.
- Gumminess: Force required to disintegrate a sample in a semi-solid state until it becomes a swallowable state that can be swallowed.
- Chewiness: Energy required to transfer a solid state of a sample into a swallowable state.
- Brittleness: Breaking or brittle property upon compression, which is represented as an intermediate peak that appears before reaching the maximum force of first compression, but may not appear depending on a sample.
- Resilience: Property in which a sample attempts to recover the original height.

As such, it was confirmed that the scaffold produced according to Example 1 had suitable compressive strength and tensile strength for the growth and differentiation of muscle cells, and as an edible scaffold used for the production of in-vitro meat, also had good texture with suitable elasticity and resilience properties and soft swallowability with suitable hardness and gumminess.

### Example 2.3 Property comparison with existing scaffolds

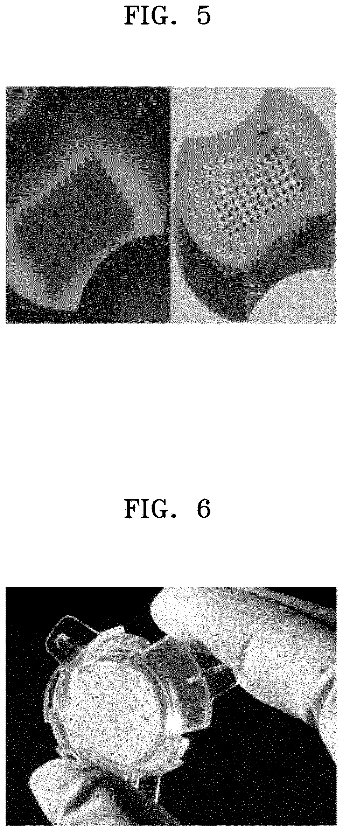

The results of comparing the properties of the previously known scaffolds (e.g., concave microwell, porous scaffold, non-porous microbead, porous microbead, etc) and the porous scaffold of Example 1 are shown in Table 6. For the properties of the previously known scaffolds, the prior art document [Materials Science & Engineering C 103 (2019) 109782] was cited. The shapes of the previously known scaffolds are shown in FIGS. 5 to 8.

**[Table 6]**

| | Concave Microwell (FIG. 5) | Porous Scaffold (FIG. 6) | Non-porous microbead (FIG. 7) | Porous Microbead (FIG. 8) | Example 1 |
|---|---|---|---|---|---|
| Cell adhesion | N/A | *** | *** | *** | ***** |
| Media diffusion to the cells | * | *** | ***** | ***** | ***** |
| Enduranc e in bioreactor s | * | * | ***** | *** | ***** |
| Cell density when fully attached | N/A | * | *** | ***** | ***** |
| Price | ***** | * | *** | * | ***** |
| Material | Scaffold-free | Plastic | Plastic/Protein | Plastic/Protein | Protein |
| Suitability for in-vitro meat productio n | Research use products only | Research use products only | Not enough yield, aggregation occurs | Not strong enough to be used in bioreactors | Great for in-vitro meat application |

As such, it was confirmed that, compared to the existing scaffolds for cell culture, the scaffold of Example 1 had high cell adhesion, excellent permeability of the culture medium, and high durability, and was made of materials suitable for the production of in-vitro meat.

### Example 3. Three-dimensional culture of cells using porous scaffold

Cells were cultured three-dimensionally by using the porous scaffold according to an embodiment.

In detail, the porous scaffold of Example 1 having a diameter of 10 mm and a thickness of 2 mm was placed on a culture dish, and 10 % mesenchymal stem cell growth medium 2 (Promocell C-28009, C-39809) was added thereto. Then, 5.0 × 10⁵ adipose-derived stem cells (ATCC PCS-500-011) were dispensed and cultured therein for 15 days for proliferation and histogenesis of the cells.

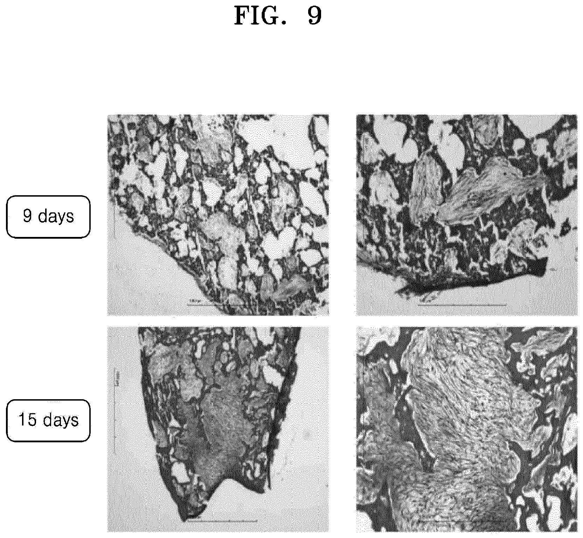

As shown in FIG. 9, it was confirmed that the growth of cells continued as the culture period elapsed; the scaffold contracted as the cells adhered to the scaffold and grew thereon; and tissues were formed as the number of the cells increased and the void volume was minimized.

### Example 4. Production of in-vitro meat by using porous scaffold

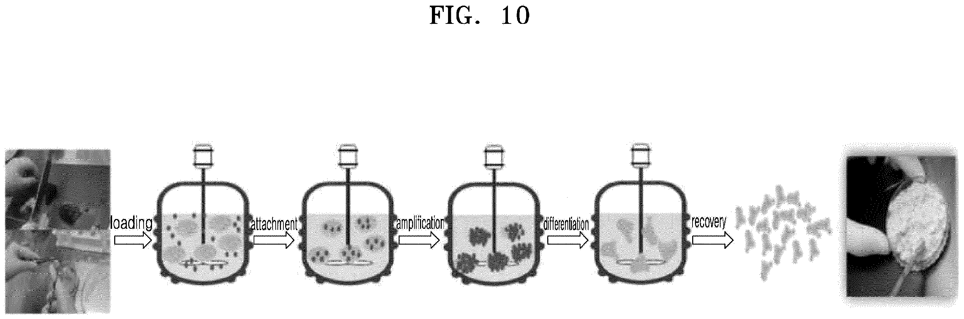

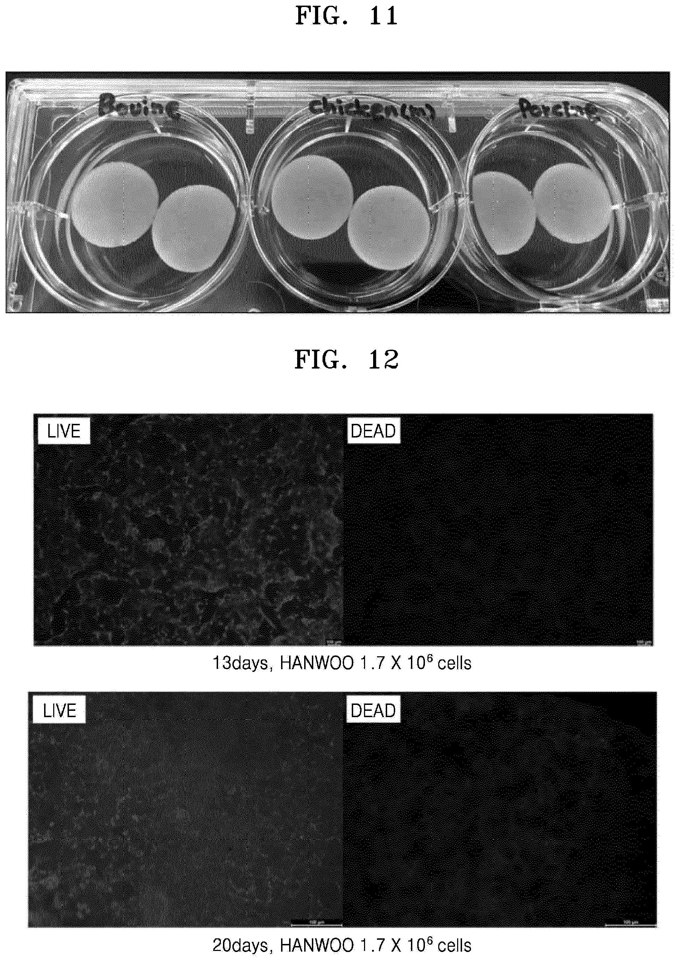

In-vitro meat was produced by using the porous scaffold of Example 1. In detail, as shown in FIG. 10, bovine muscle cells, chicken muscle cells, and pig muscle cells were respectively introduced into a bioreactor along with the scaffold of Example 1, and were collectively cultured. The results are shown in FIG. 11.

As such, it was confirmed that, when the cells and the porous scaffold were simultaneously introduced into a single incubator, the one-step mass culture system in which the adhesion, attachment, proliferation, and differentiation of the cells occur at once can be established.

### Example 4.1. Production of in-vitro beef by using porous scaffold

In a culture dish, the porous scaffold of Example 1 having a size of 25 mm × 25 mm × 2 mm, 1.7 × 10⁶ Bovine #1 cells (i.e., primary skeletal muscle cells extracted from 150 g of rump muscles of a 36-month-old female Hanwoo (Korean native cattle, dressed weight: 339 kg, quality grade 2) slaughtered by Bunong Livestock Inc. on the same day as the experiment), and each of culture media (10 % DMEM of DMEM biowest L0103-500 and 2 % DMEM of FBS biowest S1480-500) were added, and the cells were cultured for 24 hours in a shaking incubator. As a result of the culture, it was confirmed that all cells were dispensed in the porous scaffold, except for 2 % to 3 % of the residual cells.

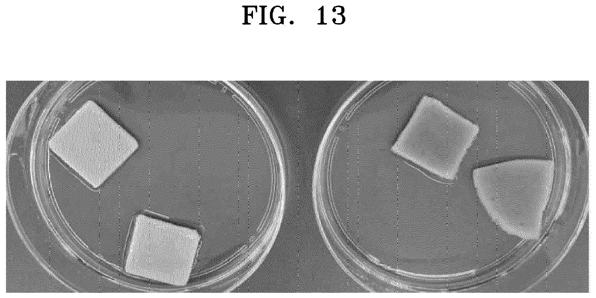

In addition, in the process of 4-week culture, the live&dead assay was performed on the 13th day and the 20th day, and the results are shown in FIG. 12. As shown in FIG. 12, it was confirmed that the cell adhesion was well achieved since dead cells were hardly observed on the scaffold, and that the cell proliferation was also well achieved on the scaffold through the increase of live cells within a week. Also, as shown in FIG. 13, as a results of visually observing the scaffold before and after the culturing, it was found that the scaffold after the culturing was changed to a pale pink color unlike the scaffold color before the culturing. Such a color change refers that proliferation of the muscle cells occurred on the scaffold.

Subsequently, in a culture dish, the porous scaffold of Example 1 having a size of 25 mm × 25 mm × 2 mm and culture media (10 % DMEM of DMEM biowest L0103-500 and 2 % DMEM of FBS biowest S1480-500) were each added. Then, 1.0 × 10⁶ Bovine #2 cells (i.e., primary skeletal muscle cells extracted from 150 g of rump muscles of a 23-month-old female Hanwoo (Korean native cattle, dressed weight: 261 kg, quality grade 2) slaughtered by Samho Livestock on the same day as the experiment) were dispensed thereon and cultured for 7 days.

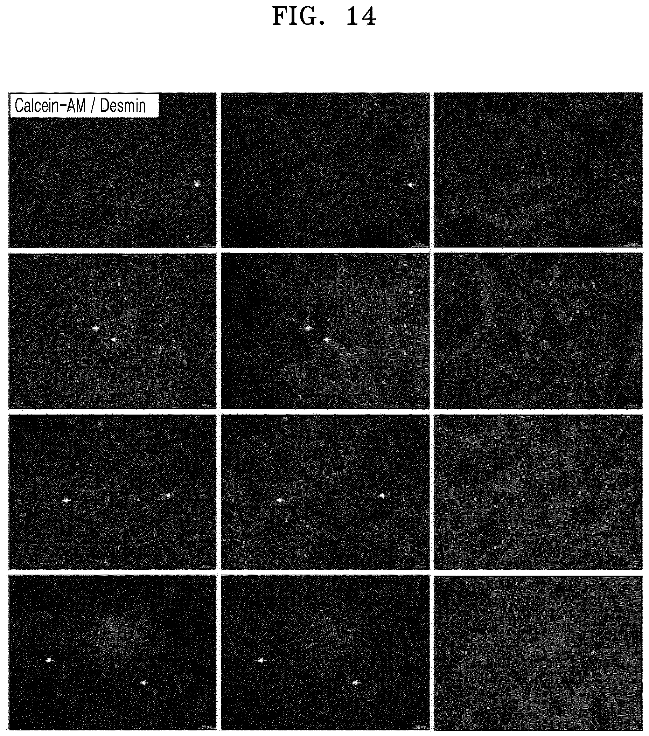

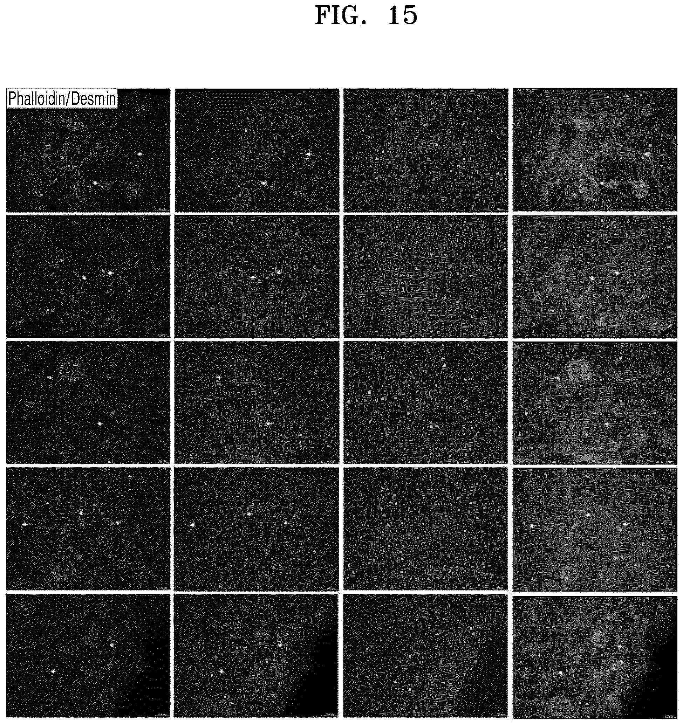

After the culture was complete, the cells were stained with each of Calcein-AM/Desmin Ab and Phalloidin/Desmin Ab, and the results are shown in FIGS. 14 and 15. Live cells and morphology of the differentiated cells were observed using calcein-AM thereof, differentiated muscle cells were observed using desmin Ab which is a muscle cell marker, and morphology of the differentiated muscle cells was observed using phalloidin. As shown in FIGS. 14 and 15, it was confirmed that the differentiation into muscle cells was well performed within the scaffold.

Accordingly, it was confirmed that in-vitro beef could be produced by culturing bovine muscle cells on the porous scaffold of Example 1.

### Example 4.2. Production of in-vitro chicken by using porous scaffold

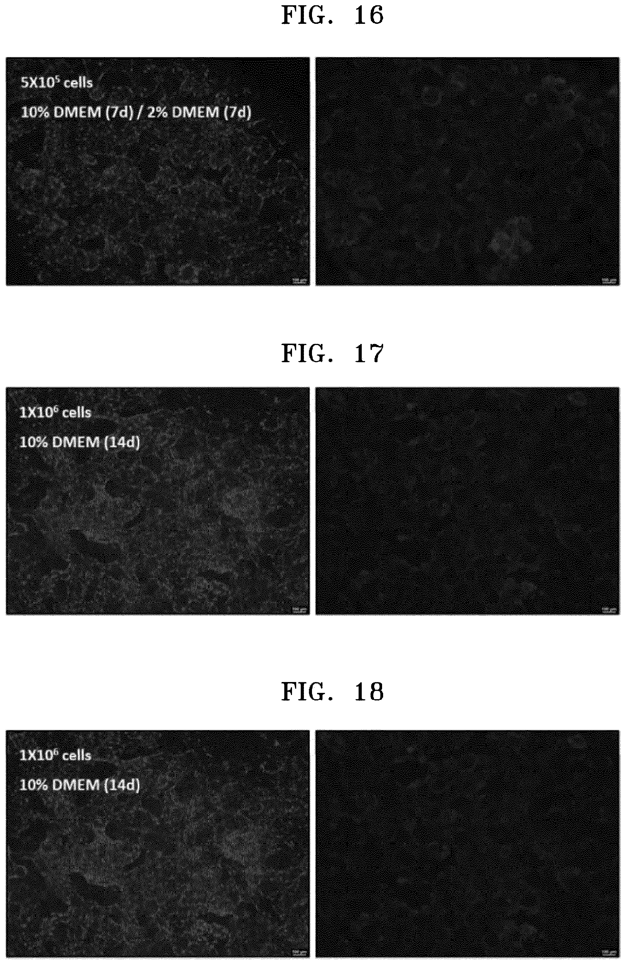

In a culture dish, the porous scaffold of Example 1 having a size of 25 mm × 25 mm × 2 mm and a culture media (10 % DMEM) were added, and 5.0 × 10⁵ chicken Cardiomyocyte #1 (i.e., primary cardiomyocytes extracted from the heart by taking embryonic body from a fertilized egg of a general layer chicken at day 9 to day 11 of hatching) were dispensed thereon and cultured for 7 days to proliferate the cells. After 7 days, the culture medium was replaced with 2% DMEM, and the cells were cultured for another 7 days to differentiate into cardiomyocytes. The live&dead assay was performed on the 14th day of the culture, and the results are shown in FIG. 16. As shown in FIG. 16, it was confirmed that the cells were generally well cultured enough to produce in-vitro meat.

Also, in a culture dish, the porous scaffold of Example 1 having a size of 25 mm × 25 mm × 2 mm and culture media (10 % DMEM and 2 % DMEM) were each added. Then, 1.0 × 10⁶ chicken Cardiomyocyte #1 (i.e., primary cardiomyocytes extracted from the heart by taking embryonic body from a fertilized egg of a general layer chicken on the 9th to 11th day of hatching by DaNagreen) were dispensed on each culture medium and cultured for 14 days. The live&dead assay was performed on the 14th day of the culture, and the results are shown in FIGS. 17 and 18. As shown in FIGS. 17 and 18, it was confirmed that the cells were generally well cultured enough to produce in-vitro meat.

Accordingly, it was confirmed that in-vitro chicken could be produced by culturing chicken muscle cells on the porous scaffold of Example 1.

Subsequently, in a culture dish, the porous scaffold of Example 1 having a size of 25 mm × 25 mm × 2 mm and culture media (10 % DMEM of DMEM biowest L0103-500 and 2 % DMEM of FBS biowest S1480-500) were added. Then, 1.0 × 10⁷ Silkie #1 cells (i.e., primary skeletal muscle cells extracted from thigh by taking embryonic body from a fertilized egg of silkie on the 9th to 11th day of hatching) were dispensed on each media and cultured for 5 weeks.

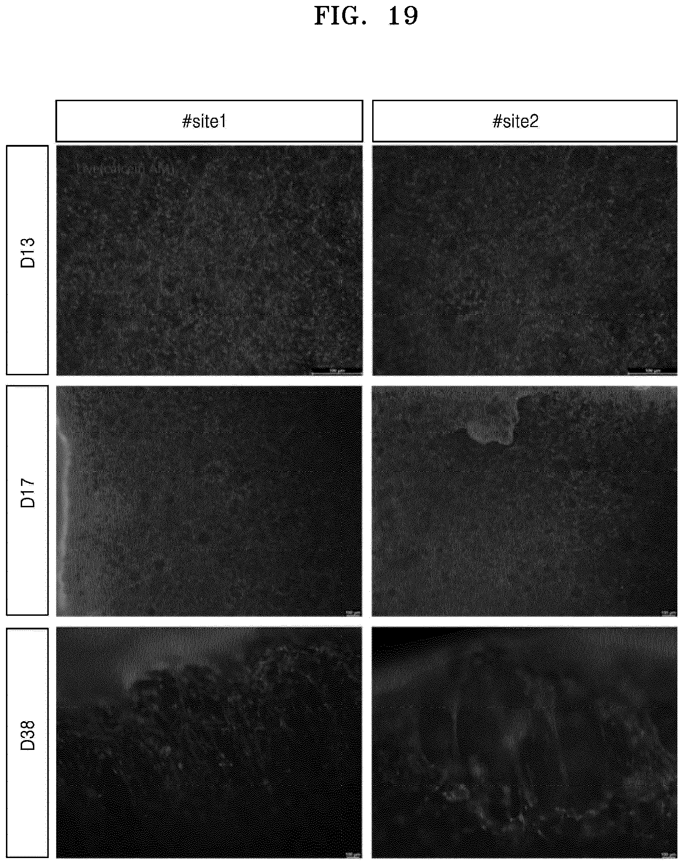

The live&dead assay was performed on the 13th day, 17th day, and 38th day of the culture, and the results are shown in FIG. 19. As shown in FIG. 19, it was confirmed that the proliferation and differentiation of the silkie muscle cells were well achieved within the scaffold. In detail, live cells were full on the scaffold at the 2nd week of the culture, and the viability of the cells was confirmed even at the 5th week of the culture.

### Example 4.3. Comparison of in-vitro beef and in-vitro chicken using porous scaffold

In a culture dish, the porous scaffold of Example 1 having a size of 25 mm × 25 mm × 2 mm and culture media (10 % DMEM of DMEM biowest L0103-500 and 2 % DMEM of FBS biowest S1480-500) were added. Then, 1.0 × 10⁶ Bovine #1 cells and 2.0 × 10⁶ Chicken #1 cells were dispensed on each cell media and cultured for 4 weeks.

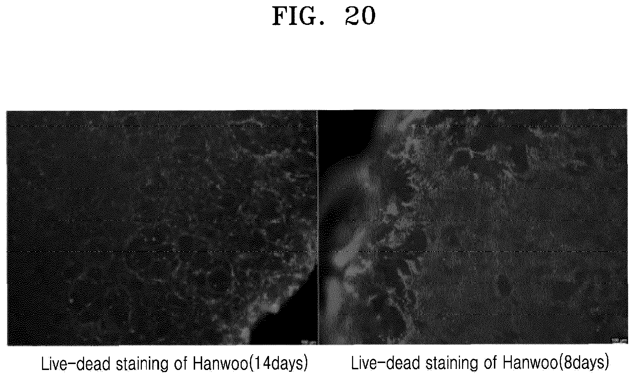

In the process of culturing, the live&dead assay was performed for the bovine cells on the 14th day and for chicken cells on the 8th day, and the results are shown in FIG. 20. As shown in FIG. 20, the chicken cells had a smaller size and a faster doubling time than the bovine cells. Thus, when cultured for the same period, it was confirmed that the scaffold for the chicken cells was filled in a much shorter period of time. It was also confirmed that, in both in-vitro meat products, the cell properties were not degraded by the scaffold, and that the in-vitro beef had different muscle fiber morphology from the in-vitro chicken.

Subsequently, as a results of tasting the in-vitro beef and the in-vitro chicken in the 3rd week and the 4th week of the in-vitro process, it was confirmed that, in the case of the in-vitro chicken, the taste and flavor of real chicken were made by only 3-week culturing of the chicken cells, and in the case of the in-vitro beef, the taste and flavor of real beef were made after 4 weeks of the bovine cell culturing was complete.

Accordingly, it was confirmed that, by using the porous scaffold of Example 1, the taste and flavor suitable for the properties of the corresponding in-vitro meat products may be exhibited.

## Claims

1. A porous scaffold comprising a plant-derived protein isolate, a porogen, an emulsifier, and a coagulant,
wherein the porous scaffold is obtained by adding the plant-derived protein isolate, the porogen, and the emulsifier to a solvent to prepare a mixture, stirring the mixture, molding and freeze-drying the mixture, wherein freeze drying comprises coating the entire surface of a frozen plant protein composition with sodium bicarbonate and then a drying the coated mixture; and adding a coagulant to the stirred mixture, wherein the porous scaffold has properties (d), (e), and (o):
(d) open porosity in a range of 40 % to 80 %;
(e) total porosity in a range of 40 % to 80 %; and
(o) pore interconnectivity in a range of 80 % to 99.9 %.

2. The porous scaffold of claim 1, wherein the porous scaffold has any one or more properties selected from (a) to (c):
(a) compressive strength in a range of 80 kPa to 150 kPa;
(b) tensile strength in a range of 0.392 N to 0.686 N (40 gf to 70 gf), yield strength in a range of 18 kPA to 32 kPA, and yield elongation in a range of 10 % to 20 %; and
(c) hardness in a range of 3.92 N to 9.81 N (0.4 kgf to 1.0 kgf), cohesiveness in a range of 0.6 to 1.0, springiness in a range of 0.8 to 1.2, gumminess in a range of 0.3 to 0.7, and chewiness of 0.3 to 0.7.

3. The porous scaffold of claim 1, wherein the porous scaffold has a pore size in a range of 50 µm to 400 µm.

4. The porous scaffold of claim 1, wherein the plant-derived protein isolate is a protein isolated from at least one selected from the group consisting of nut, bean, soybean, mung bean, kidney bean, pea, mushroom, vegetable, grain, and a combination thereof.

5. The porous scaffold of claim 1, wherein the porogen includes at least one selected from the group consisting of agar, salt, calcium chloride, sodium carbonate, paraffin, polyethylene glycol, gelatin, sucrose, and a combination thereof.

6. The porous scaffold of claim 1, wherein the emulsifier or the solvent includes at least one selected from the group consisting of glycerin, propylene glycol, monoglyceride, diglyceride, lecithin, soybean phospholipid, and a combination thereof.

7. The porous scaffold of claim 1, wherein the coagulant includes at least one selected from the group consisting of glucono-delta-lactone, calcium chloride, calcium sulfate, magnesium chloride, and a combination thereof.

8. The porous scaffold of claim 1, wherein cells are dispensed in the porous scaffold.

9. The porous scaffold of claim 1, wherein the porous scaffold is for the production of in-vitro meat.

10. A method of producing a porous scaffold, wherein the porous scaffold has properties (d), (e), and (o):
(d) open porosity in a range of 40 % to 80 %;
(e) total porosity in a range of 40 % to 80 %; and
(o) pore interconnectivity in a range of 80 % to 99.9 %,
the method comprising:
adding a plant-derived protein isolate, a porogen, and an emulsifier to a solvent to prepare a mixture and stirring the mixture to produce a stirred mixture; molding and freeze-drying the mixture, wherein freeze drying comprises coating the entire surface of a frozen plant protein composition with sodium bicarbonate and then a drying the coated mixture; and adding a coagulant to the stirred mixture to form a scaffold.

11. In-vitro meat comprising:
the porous scaffold of claim 1; and
a cell dispersed in the porous scaffold.

12. A method of producing in-vitro meat, the method comprising dispensing a cell in the porous scaffold of claim 1 and culturing the cell three-dimensionally to form a three-dimensional cell aggregate.

13. The method of claim 12, wherein the cell is at least one selected from the group consisting of a stem cell, a muscle cell, an adipocyte, and a combination thereof.

14. An edible product comprising the in-vitro meat of claim 11.
